(19) **Europäisches Patentamt European Patent Office Office européen des brevets**

(11) **EP 2 806 785 B1**

(12) **EUROPEAN PATENT SPECIFICATION**

(45) Date of publication and mention of the grant of the patent:
**21.09.2016 Bulletin 2016/38**

(21) Application number: **13711739.6**

(22) Date of filing: **22.01.2013**

(51) Int Cl.:
***A61B 5/00*** *(2006.01)*

(86) International application number:
**PCT/IB2013/050545**

(87) International publication number:
**WO 2013/111053 (01.08.2013 Gazette 2013/31)**

(54) **AN APPARATUS FOR OPTICAL ANALYSIS OF AN ASSOCIATED TISSUE**

VORRICHTUNG ZUR OPTISCHEN ANALYSE EINES ZUGEHÖRIGEN GEWEBES

APPAREIL POUR L'ANALYSE OPTIQUE D'UN TISSU ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.01.2012 US 201261591320 P**

(43) Date of publication of application:
**03.12.2014 Bulletin 2014/49**

(73) Proprietor: **Koninklijke Philips N.V.
5656 AE Eindhoven (NL)**

(72) Inventors:
- **HENDRIKS, Bernardus Hendrikus Wilhelmus
  NL-5656 AE Eindhoven (NL)**
- **SPLIETHOFF, Jarich Willem
  NL-5656 AE Eindhoven (NL)**
- **NACHABE, Rami
  NL-5656 AE Eindhoven (NL)**
- **RUERS, Theodoor Jacques Marie
  NL-5656 AE Eindhoven (NL)**
- **LUCASSEN, Gerhardus Wilhelmus
  NL-5656 AE Eindhoven (NL)**
- **HORIKX, Jeroen Jan Lambertus
  NL-5656 AE Eindhoven (NL)**
- **MUELLER, Manfred
  NL-5656 AE Eindhoven (NL)**
- **VAN DER VOORT, Marjolein
  NL-5656 AE Eindhoven (NL)**

(74) Representative: **Steffen, Thomas
  Philips Intellectual Property & Standards
  High Tech Campus 5
  5656 AE Eindhoven (NL)**

(56) References cited:
**WO-A1-2010/144081     US-A1- 2011 112 435**

- **Ruplal Choudhary: "RAPID ESTIMATION OF LYCOPENE CONCENTRATION IN WATERMELON AND TOMATO SAMPLES BY FIBER OPTIC VISIBLE SPECTROSCOPY", , 31 December 2004 (2004-12-31), XP055064392, Retrieved from the Internet: URL:http://digital.library.okstate.edu/etd /umi-okstate-1092.pdf [retrieved on 2013-05-28]**

**Description**

FIELD OF THE INVENTION

The present invention relates to an apparatus for optical analysis of an associated tissue, and more specifically to an apparatus, a method and a computer program for determination of a parameter indicative of tissue type of the associated tissue.

BACKGROUND OF THE INVENTION

During interventions in the field of surgical oncology and prostate cancer treatment including associated structures, it is important to be able to discriminate pathological tissue from normal tissue in order for instance to ensure that the treatment is performed on the correct location. Although differences in blood content will likely provide possibilities to discriminate certain structures of the prostate organ, they are not always sufficient for instance in early stage prostate cancer.

WO 2010/144081 A1 discloses apparatuses for detecting Raman scattered light from a sample, including *in vivo* biological tissue samples. The apparatuses include a sample probe comprising an optical fiber, the optical fiber adapted to deliver excitation light along the fiber to the sample and to collect the Raman scattered light from the sample along the same fiber; an optical module coupled to the sample probe, the optical module adapted to direct the excitation light and the Raman scattered light and to separate the excitation light from the Raman scattered light; and a detector module coupled to the optical module, the detector module adapted to detect the Raman scattered light from the sample over a particular spectral region, including about 2000 cm$^{-1}$ or less.

An apparatus which could be beneficial for discriminating certain tissue types within the prostate organ would be advantageous.

Hence, an improved apparatus which could be beneficial for discriminating certain tissue types within the prostate organ would be advantageous, and in particular a more simple, precise, effective and reliable apparatus would be advantageous.

SUMMARY OF THE INVENTION

In particular, it may be seen as an object of the present invention to provide an apparatus, a method and a computer program which could be beneficial for discriminating certain tissue types within the prostate organ hat solves the above mentioned problems of the prior art with being simple, precise, effective and versatile.

It is a further object of the present invention to provide an alternative to the prior art.

Thus, the above described object and several other objects are intended to be obtained in a first aspect of the invention by providing an apparatus for optical analysis of an associated tissue, as defined by the independent claim 1. The dependent claims define advantageous embodiments.

The invention is particularly, but not exclusively, advantageous for obtaining a simple, precise, effective and reliable apparatus for optical analysis of an associated tissue, which may be employed for determination of a first parameter indicative of concentration of lycopene in an associated tissue.

It is understood that the first parameter being indicative of a concentration of lycopene in said associated tissue may in particular be indicative of the specific concentration of lycopene, such as the specific ("partial") concentration of lycopene within a mixture of chromophores, such as a mixture of carotenoids. For example, the first parameter may be indicative of the specific concentration of lycopene. The specific concentration of lycopene may be determined in an associated tissue which comprises a total concentration of carotenes being higher than the specific ("partial") concentration of lycopene.

The problem of discriminating certain tissue types within the prostate organ is solved by the present invention which enables finding an additional distinguishing feature - namely concentration of lycopene - that can enable differentiation of various normal and/or diseased tissue structures in the prostate organ system, i.e., in the prostate organ and associated structures.

The invention is based on the insight made by the present inventors that lycopene may be measured via an interventional device. Measuring the concentration of lycopene might be useful, since such measure of concentration of lycopene may serve as a discriminating feature, such as for discriminating between tissue types, such as determining whether a prostate tissue is normal tissue or tumour tissue.

The gist of the invention may be seen as providing an apparatus which enables measuring lycopene, e.g., in organs in a minimally invasive manner.

The invention provides a technical solution to a technical problem, and may assist a physician in reaching a diagnosis or treating a patient.

Lycopene is known in the art. Lycopene is a member of the carotenoid family. Carotenoids are a collective term for a large group of molecules, of which more than hundreds of variants exist. The two most common variants are β-carotene and lycopene which can be found in carrots and tomatoes, respectively. Both of these carotenoids may, to some extent act as a sun blocker when present in the skin since the absorption is most pronounced in the UV and blue wavelength region. However, carotenoids are not only found in the skin but also in the blood stream, aorta and the macula lutea.

It is noted, that while prior art exist, such as WO 2010/144081 A1, which may allegedly be able to employ an interventional device and determine a parameter in an associated tissue which may be indicative of the presence of, for instance a so-called "C=C bond", which may be due to carotenoid, such prior art still does not enable determination of a parameter indicative of a concentration of lycopene in an associated tissue, since such prior art is not able to resolve which carotenoid is involved.

When referring to concentration of lycopene, it is understood that the concentration of lycopene is to be measured in amount per volume, such as molar concentration (i.e., amount of lycopene divided by volume). It is further understood that the concentration of a chromophore, such as lycopene, in an associated tissue may be determined within an accuracy of within +/- 30 %, such as within +/- 25 %, such as within +/- 20 %, such as within +/- 18 %, such as within +/- 16 %, such as within +/- 14 %, such as within +/- 12 %, such as within +/- 10 %, such as within +/- 9 %, such as within +/- 8 %, such as within +/- 7 %, such as within +/- 6 %, such as within +/- 5 %, such as within +/- 4 %, such as within +/- 3 %, such as within +/- 2 %, such as within +/- 1 %.

According to an embodiment of the invention, there is provided an apparatus wherein the determination of the first parameter enables discriminating between normal and tumor tissue, such as normal prostate tissue and tumor tissue. This may be advantageous in that the apparatus may enable measuring the concentration of lycopene in tissue in a prostate and thus enable determining whether the tissue is normal tissue or tumor tissue.

Light is to be broadly construed as electromagnetic radiation comprising wavelength intervals including visible, ultraviolet (UV), near infrared (NIR), infrared (IR), x-ray. The term optical is to be understood as relating to light.

An optical spectrum is understood to be information related to a plurality of wavelengths of light, such as an intensity parameter, an absorption parameter, a scattering parameter or a transmission parameter given for a plurality of wavelengths of light. A continuous spectrum represents spectral information, but it is further understood, that information related to light at discrete wavelengths may represents an optical spectrum.

A spectrometer is understood as is common in the art. It is understood, that the spectrometer comprises means for selecting wavelengths, such as transmission filters or gratings. Alternatively, wavelength specific light sources, such as light emitting diodes or LASERs, may be used or wavelength specific optical detectors may be used. A spectral filtration may occur at different places in the system, for instance it may occur between the second light source and the interventional device, it may occur in the interventional device, or it may occur between the interventional device and the optical detector.

An interventional device is generally known in the art, and may include any one of an endoscope, a catheter, a biopsy needle. An interventional device may in general be understood to be an elongated probe, such as being suitable for being used in minimal invasive interventions. An interventional device may in general be understood to be suitable for insertion into body openings, body cavities and/or animal or human organs, such as the prostate or liver. An interventional device may be understood to be an elongated probe having a length of at least 1 cm, such as at least 2 cm, such as at least 3 cm, such as at least 4 cm, such as at least 5 cm, such as at least 6 cm, such as at least 7 cm, such as at least 8 cm, such as at least 9 cm, such as at least 10 cm, such as at least 15 cm, such as at least 20 cm, such as at least 25 cm, such as at least 30 cm, such as at least 50 cm, such as at least 75 cm, such as at least 100 cm, such as at least 125 cm, such as at least 150 cm, such as at least 175 cm, such as at least 200 cm. An interventional device may be understood to be an elongated probe having a diameter of less than 5.0 cm, such as less than 4.5 cm, such as less than 4.0 cm, such as less than 3.5 cm, such as less than 3.0 cm, such as less than 2.5 cm, such as less than 2.0 cm, such as less than 1.5 cm, such as less than 1.0 cm, such as less than 0.5 cm.

According to an embodiment of the invention, there is provided an apparatus wherein the processor is further arranged for determining from the first parameter a second parameter being indicative of a tissue type. The apparatus according to this embodiment may be seen as simple in that it enables procurement of measured data representative of an optical spectrum, and furthermore enables extraction of information from the measured data for assigning a parameter to the associated tissue. In other words, the apparatus enables determining a first parameter being indicative of a concentration of lycopene in said associated tissue, and furthermore enables determining from the first parameter a second parameter being indicative of a tissue type, i.e., the apparatus enables measuring within an associated tissue (such as *in vivo* measurements within a prostate) and assigning a parameter indicative of tissue type (such as normal or tumour) of the same tissue. It is noted, that this embodiment is based on the insight, that determination of a concentration of lycopene in certain tissues, such as the prostate tissue, may be indicative of the state of the tissue (i.e., being normal tissue or tumour tissue). An advantage of this embodiment is thus, that it enables using a relatively simple apparatus for carrying out a minimally invasive measurement which may yield a parameter being indicative of the state of an organ, such as the prostate.

It is understood that discrimination may include discrimination between tissue conditions, such as discrimination between normal tissue and tumour tissue. This may be relevant in order to ensure that the treatments in the field of oncology are performed on the correct location. For instance ablation of a small tumour lesion in the prostate requires accurate placement of the ablation needle tip. Image guidance by for instance X-ray or ultrasound can provide valuable feedback but these means of navigation do not provide real time tissue feedback from the tip of the needle. This makes targeting small lesions difficult with these techniques. Another advantage of the invention may be that it enables more reliable discrimination between tissue types, since it enables the determination and use of a new discriminative feature, namely the concentration of lycopene.

According to an embodiment of the invention, there is provided an apparatus wherein the apparatus is arranged for obtaining measured data representative of the optical spectrum, wherein the optical spectrum is a Diffuse Reflectance Spectrum. Diffuse Reflectance Spectroscopy (DRS) is known in the art. DRS may be advantageous for determining a concentration of one or more optically absorbing substances, such as lycopene, even in complex samples, such as an associated tissue where several chromophores may be present (e.g., lycopene, haemoglobin, etc.). In general, DRS may work by determining the concentration of optically absorbing substances (chromophores) from the attenuation of incident light, and DRS might also take into account the scattering of the sample, such as the scattering of the associated tissue. For example, a concentration of lycopene may be determined based on the absorption coefficient of lycopene. Another possible advantage of DRS may be that since diffusive photons are employed, a relatively large region of the associated tissue may be probed, even for a relatively small interventional device. Probing a relatively large region may be seen as advantageous, since it reduces a risk that a small artifact dominates the information retrieved from the probed region. Furthermore, DRS may be seen as advantageous because it allows for a relatively simple apparatus and technique.

In the present application, it is shown that lycopene has a specific absorption profile. With DRS it is possible to utilize these absorption effects to determine the concentration of lycopene, i.e., to determine the concentration of a *specific* carotenoid, such as lycopene.

According to an embodiment of the invention, there is provided an apparatus wherein the exit position and the entry position being positioned with sufficient relative distance for Diffusive Reflectance Spectroscopy (DRS) to be performed. Diffusion theory may require a certain minimum distance $d$ between the center-to-center distance separation between the exit position of a first (emitting) guide and the entry position of any one of a second (collecting) guide and a third (collecting) guide, a fourth (collecting) guide, etc. The distance $d$ is important for DRS because this distance determines the accuracy and also the spatial depth of the area probed by DRS. Roughly a depth of d/2 is probed. If two light guides are present the influence of artifacts in optical tissue characterization can be circumvented by choosing the distance $d$ sufficiently large so that the probed volume is at a sufficiently large with a probing depth approximately equal to half the distance between the fibers, i.e., a depth of $d$/2.

In particular embodiments, the center-to-center distance separation $d$ between the center-to-center distance separation between the exit position (of a first (emitting) guide) and the entry position (of any one of a second (collecting) guide) may be in the millimeter range, such as at least 0.1 mm, such as at least 0.5 mm, such as at least 1 mm, such as at least 2 mm, such as 2.5 mm, such as at least 3 mm, such as at least 5 mm, such as at least 10 mm.

All guides may be low-OH fibers of core diameters in the micron range, such as core diameter of 200 microns. Fibers containing low-OH, sometimes also called VIS-NIR fibers, are typically suitable for the visible (VIS) and near infrared (NIR) part of the optical spectrum.

It is also noted that if more than two light guides are present (i.e., more than one exit position and/or more than one entry position), different volumes can be probed at different distances in front of the interventional device. In this case, possible effects (such as from local tissue artifacts) which may have an impact on the optical signal may be considered an additional source of information that may help the physician (in interpreting the data) rather than merely being considered an artifact.

According to an embodiment of the invention, there is provided an apparatus, wherein the processor is further arranged for determining one or more third parameters, wherein each of the one or more third parameters are indicative of a concentration of a chromophore other than lycopene in said associated tissue. It is understood that the one or more third parameters may be a single number or a set comprising a plurality of numbers. For example, the one or more third parameters may be a single number being wherein the number is indicative of a concentration of a chromophore other than lycopene in said associated tissue. In another example, the one or more third parameters may be a set comprising a plurality of numbers wherein each number is indicative of a concentration of a chromophore other than lycopene in said associated tissue. In particular embodiments, the chromophore other than lycopene may be chosen from the group comprising: beta-carotene, collagen, elastin, bile, oxygenated haemoglobin, deoxygenated haemoglobin, billirubin, lipids, and water. An advantage of determining one or more third parameters, wherein each of the one or more third parameters are indicative of a concentration of a chromophore other than lycopene in said associated tissue, may be that more information about the associated tissue may thus be achieved.

[2]According to an embodiment of the invention, there is provided an apparatus wherein the processor is further arranged for determining a scattering parameter based on the measured data. A possible advantage of this may be that determination of a scattering parameter renders it possible to take the scattering parameter into account. For example, an algorithm for disentangling contributions from different optically active constituents, such as chromophores, in an associated tissue may not be able to correctly disentangle the contributions and correctly quantify the constituents if scattering is present in the associated tissue, unless the algorithm determines the scattering parameter and takes it into account.

According to an embodiment of the invention, there is provided an apparatus further comprising an interventional device, the interventional device comprising a first guide for guiding photons from the light source to an exit position on a distal end of the interventional device, the photons being emittable from the exit position, and a second guide for guiding photons from an entry position on the distal end of the interventional device and to the optical detector. The first guide and the second guide may be two separate guides which are spatially distanced from each other. Each of the first and second are understood to be light guides, such as optical fibres, such as optical waveguides. An advantage of having a first and second guide may be that it enables guiding photons to and from, respectively, the exit and entry positions, in order to bring the photons from the light source, respectively, to the optical detector.

According to an embodiment of the invention, there is provided an apparatus wherein the exit position and the entry position are spatially separated and spatially oriented so that, upon positioning the distal end of the interventional device adjacent to the associated tissue, the entry position is not intersected by ballistic photons emitted from the exit position, when the distal end of the interventional device is placed adjacent the associated tissue. It is understood that the entry position is not intersected by ballistic photons emitted from the exit position, at least from a practical point of view. For all practical purposes, the number of ballistic photons hitting the entry position is non-zero but negligible.

Ballistic photons are construed as photons which move in straight lines without being scattered more than once, such as a photon used for imaging which is scattered once on the imaged object.

Diffusive photons are photons which experience multiple, scattering events, such as multiple random scattering events. The scattering events may be elastic, such as Rayleigh scattering, or inelastic, such as Raman scattering. Absorption of photons emitted at the exit position may take place at certain wavelengths giving rise to particular absorption bands being visible in the spectrum of the diffusive photons being collected at the entry position.

By arranging the entry and exit positions as described, a large majority of photons collected at the entry position will be diffusive photons which have traversed a relatively long and non-straight path between the exit and entry position. In total, when using a large number of photons, as will generally be the case, the information collected together with the photons collected at the entry position will be dependent on a region in front of the interventional device, the region being traversed by the diffusive photons emitted at the exit position.

According to an embodiment of the invention, there is provided an apparatus wherein the exit position and the entry position are spatially separated and spatially oriented so that, upon positioning the distal end of the interventional device adjacent to the associated tissue the photons emittable at the exit position and subsequently collectable at the entry position are diffusive photons which experience multiple scattering events. An advantage of collecting diffusive photons may be that in general they have traversed a larger region, compared to ballistic photons.

It is noted that the terms "diffusive photons" or "ballistic photons" do not relate to properties of the photons as such, but instead relates to the path the photons take between the exit and entry position when emitted from the exit position when the distal end of the interventional device is placed in front of the associated tissue.

According to an embodiment of the invention, there is provided an apparatus wherein photons emitted from the exit position are non-focused. The photons may initially after exiting the exit position on the distal end of the interventional device constitute paraxial or diverging rays, or they may otherwise be non-focused. It is understood that in the present context, the photons exiting the exit position on the distal end of the interventional device are considered non-focused if they are not focused within a distance comparable to a spatial scale of the first region. A possible advantage of this is that the energy is divided over a broader area of the adjacent associated tissue due to the defocusing, and as a result there is less risk of damaging the adjacent associated tissue.

According to an embodiment of the invention, there is provided an apparatus wherein the apparatus further comprises a database, which database is operably connected to the processor. An advantage of this may be, that the processor may access data stored in the database, which data may be beneficial for determining from the measured data a first parameter being indicative of a concentration of lycopene in said associated tissue.

According to a further embodiment of the invention, there is provided an apparatus wherein the database comprises predetermined data representative of one or more optical spectra, wherein one predetermined data, such as one predetermined optical spectrum, is representative of an optical spectrum of lycopene. Having predetermined data representative of an optical spectrum stored in the database may be beneficial for determining from the measured data a first parameter being indicative of a concentration of lycopene in said associated tissue, and determining from the first parameter a second parameter being indicative of a tissue type. The predetermined data may be representative of spectra of a tissue type, or the predetermined data may be representative of an optical spectrum of a chromophore expected to be in the associated tissue, which may be useful, e.g., as an input parameter in a mathematical model.

In a particular embodiment, the database comprises data regarding levels of lycopene in different tissue types. An advantage of this may be that it enables determining from the first parameter a second parameter being indicative of a tissue type, e.g., by comparing the measured lycopene concentration with standard values of lycopene within different tissue types, thus utilizing the discriminating power of the concentration of lycopene.

In an embodiment of the invention, there is provided an apparatus further comprises any one of: a light source for providing therapeutic light and/or an ultrasound unit. A possible advantage of providing a therapeutic light source is that it enables therapy using light. An advantage of providing an ultrasound unit may be that it enables ablation, such as radio frequency ablation or imaging.

According to a second aspect of the invention, the invention further relates to a method for optical analysis of an associated tissue, as defined by the independent method claim. The dependent claims define advantageous embodiments.

This second aspect of the invention is particularly, but not exclusively, advantageous in that the method according to the present invention may be implemented by the apparatus according to the first aspect, so as to enable a relatively simple apparatus and method for obtaining the concentration of lycopene in a minimally invasive manner.

According to an embodiment of the invention, there is provided a method wherein the determination of the first parameter includes any one of:

- fitting the measured data to a mathematical model,
- accessing a look-up-table comprising one or more predetermined optical spectra, and
- performing multivariate analysis.

In a special embodiment of a method according to the invention, the embodiment comprises administering lycopene to the patient before measuring. In this way the concentration of lycopene may be enhanced during measurement and determination of the tissue. Lycopene may be administered by putting the subject, such as a patient, on a lycopene rich diet, such as a diet comprising eating tomatoes.

According to a third aspect of the invention, the invention further relates to a computer program product being adapted to enable a computer system comprising at least one computer having data storage means associated therewith to operate a processor arranged for carrying out the method according to the second aspect.

The first, second and third aspect of the present invention may each be combined with any of the other aspects. These and other aspects of the invention will be apparent from and elucidated with reference to the embodiments described hereinafter.

BRIEF DESCRIPTION OF THE FIGURES

The apparatus, method and a computer program for determination of a parameter indicative of tissue type of the associated tissue according to the invention will now be described in more detail with regard to the accompanying figures. The figures show one way of implementing the present invention and is not to be construed as being limiting to other possible embodiments falling within the scope of the attached claim set.

FIG 1       shows a diagrammatic depiction of an apparatus according to an embodiment of the invention,
FIG 2       shows an interventional device according to an embodiment of the invention,
FIG 3       shows spectra of the absorption coefficient of lycopene, β-carotene (beta-carotene) oxygenated haemoglobin (Hb) and deoxygenated haemoglobin (HbO2),
FIG 4       shows a DRS measurement of normal prostatic tissue and fitting with standard chromophores (range: 400-1600nm),
FIGS 5-6    show the result of fitting with only default chromophores, respectively, including β-carotene (beta-carotene) in the fitting,
FIG 7       shows a standard fit for a wavelength range of 400-600 nm,
FIG 8       shows a fit with β-carotene (beta-carotene) included into the model for a wavelength range of 400-600 nm,
FIG 9       shows a fit with β-carotene (beta-carotene) and lycopene included into the model for a wavelength range of 400-600 nm,
FIG 10      is a flowchart of a method according to the invention, and
FIG 11      shows an overview of biopsies obtained for the studies for the present application.

DETAILED DESCRIPTION OF AN EMBODIMENT

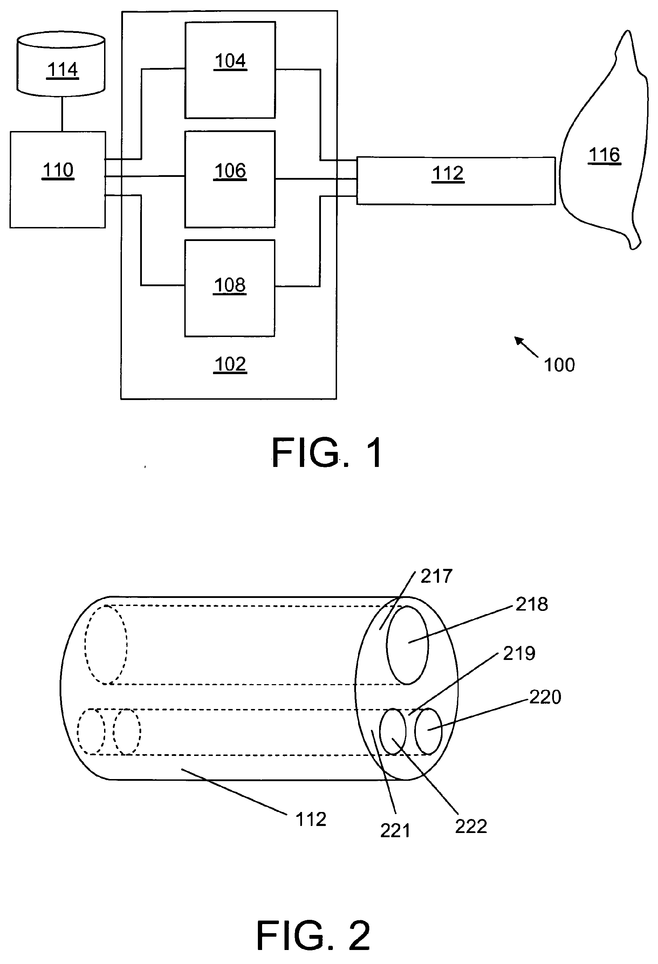

FIG 1 shows a diagrammatic depiction of an apparatus according to an embodiment of the invention comprising a spectrometer 102 comprising a light source 104, a first optical detector 106, an optional second optical detector 108 and an interventional device 112, where the interventional device 112 has one or more guides, such as optical elements, such as optical waveguides, capable of guiding light from the light source 104 to a distal end of the interventional device so as to emit the light at the distal end of the interventional device, and furthermore capable of guiding light back from the distal end of the interventional device to the first optical detector 106 and/or second optical detector 108. The light guides enable light to enter an associated tissue 116, such as a prostate tissue, and the light guides further enable light exiting the associated tissue to be collected and led to the optical detector. The apparatus thus enables procurement of measured data representative of an optical spectrum of the associated tissue 116. The optical detectors 106, 108 may be controlled by processor 110 so as to acquire the measured data. The processor may have access to a database 114.

In a specific embodiment, the apparatus is further arranged to access the database 114, where the database is comprising information regarding various tissue types, such as standard values of concentrations of certain chromophores within various tissue types, and identify which tissue type or tissue types the associated tissue is most likely to comprise, and wherein the identification is based on the first parameter. According to a particular embodiment, the processor 110 is arranged for determining from the first parameter a second parameter being indicative of a tissue type. An advantage of this is that valuable information regarding the tissue type might be obtained this way.

The invention may in a particular embodiment be described as comprising a console (comprising, e.g., a spectrometer 102 comprising a light source 104, a first optical detector 106), and an interventional device 112, such as an elongated optical probe and the console being connected to a processor 110 which is arranged for accessing an algorithm which enables determining the lycopene concentration in front of the elongated device (based on the measurements of the associated tissue in front of the elongated device). In a particular embodiment, the device may further translate the concentration of lycopene into a tissue type, such as by determining a second parameter being indicative of tissue type.

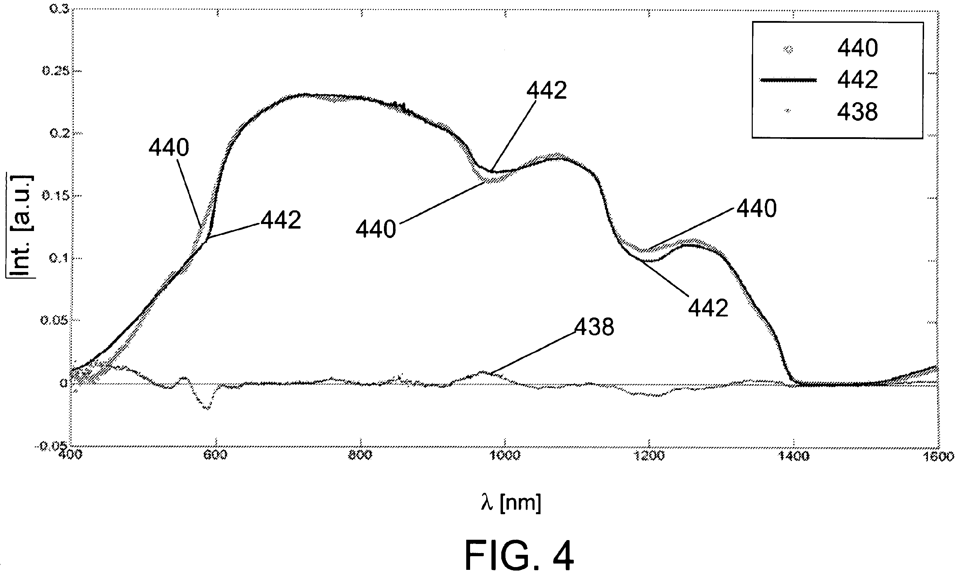

It will be shown (in FIGS 4-9) that absorption features of lycopene are present near the blue part of the spectrum (400-500 nm). Hence, in a particular embodiment, the apparatus is adapted for obtaining measured data representative of an optical spectrum of the associated tissue, and correcting the measured data for stray light, such as stray light in the spectrometer.

FIG 2 shows a perspective illustration of an embodiment of an interventional device 112, which interventional device comprises a first guide 217, a second guide 219 and a third guide 221. The figure shows an exit position 218 on distal end of the first guide 217, an entry position 220 on a distal end of the second guide 219, and an entry position 222 on a distal end of the third guide 221. The drawing is not to scale. The first, second and third guide are understood to be light guides, such as optical fibers, such as optical waveguides. In a specific embodiment, the apparatus comprises a light source 104 in the form of a halogen broadband light source with an embedded shutter, an interventional device 112 with three guides and two optical detectors 106, 108 that can resolve light in different wavelength regions, such as substantially in the visible and infrared regions of the wavelength spectrum respectively, such as from 400 nm to 1100 nm and from 800 nm to 1700 nm respectively. The apparatus may furthermore comprise a filter that rejects light for wavelengths below 465 nm which filter may be mounted in front of the optical detectors 106, 108 to reject second order light at the optical detectors. The interventional device 112 has a first guide 217 connected to the light source, the second guide 219 connected to the first optical detector 106 and the third guide 221 connected to the second optical detector 108.

Algorithm

In this study, the acquired spectra were fitted using a custom made Matlab 7.9.0 (Mathworks, Natick, MA) algorithm. In this algorithm, a widely accepted analytical model was implemented, namely the model introduced by the reference [1], which is hereby incorporated by reference in entirety. The input arguments for the model of the reference [1] are the absorption coefficient $\mu_\alpha(\lambda)$, the reduced scattering coefficient $\mu_s'(\lambda)$ and the center-to-center distance between the emitting and collecting fibers at the tip of the probe. For a complete description of the diffusion theory model, we refer to [1]. In the following part, the model will be explained briefly. The used formulas are mainly based on work of Nachabé *et al.*, and reference is thus made to [2] which is hereby incorporated by reference in entirety, and furthermore reference is made to [3] which is hereby incorporated by reference in entirety.

Scattering

A double power law function can be used to describe the wavelength dependence of the reduced scattering, where the wavelength $\lambda$ is expressed in nm and is normalized to a wavelength value of $\lambda_0$ =800 nm. The parameter $\alpha$ corresponds to the reduced scattering amplitude at this specific wavelength.

$$\mu_s'(\lambda) = a\left[\rho_{MR}\left(\frac{\lambda}{\lambda_0}\right)^{-b} + (1-\rho_{MR})\left(\frac{\lambda}{\lambda_0}\right)^{-4}\right]\left[cm^{-1}\right] \qquad (Eq.1)$$

In this equation the reduced scattering coefficient is expressed as the sum of Mie and Rayleigh scattering where $\rho_{MR}$ is the Mie-to-total reduced scattering fraction. The reduced scattering slope of the Mie scattering is denoted b and is related to the particle size.

Absorption

For a homogeneous distribution of absorbers, the total light absorption coefficient $\mu_\alpha(\lambda)$ can be computed as products of the extinction coefficients and volume fraction of the absorbers:

$$\mu_a^{Total} = f_1\mu_a^1 + f_2\mu_a^2 + f_3\mu_a^3 + \dots \qquad (Eq.2)$$

Instead of modeling the absorption coefficient $\mu_\alpha(\lambda)$ as the sum of absorption coefficients weighted by the respective concentrations of the four chromophores of interest, it was decided to express the tissue absorption coefficient as

$$\mu_a^{Tissue}(\lambda) = C(\lambda)\upsilon_{Blood}\mu_a^{Blood}(\lambda) + \upsilon_{WL}\mu_a^{WL}(\lambda)\left[cm^{-1}\right] \qquad (Eq.3),$$

where $\mu_a^{Blood}(\lambda)$ corresponds to the absorption by blood and $\mu_a^{WL}(\lambda)$ corresponds to absorption by water and lipid together in the probed volume. The volume fraction of water and lipid is $v_{WL} = [Lipid] + [H_2O]$, whereas $\upsilon_{Blood}$ represents the blood volume fraction for a concentration of hemoglobin in whole blood of 150 mg/ml.

The factor C is a wavelength dependent correction factor that accounts for the effect of pigment packaging and alters for the shape of the absorption spectrum. This effect can be explained by the fact that blood in tissue is confined to a very small fraction of the overall volume, namely blood vessels. Red blood cells near the center of the vessel therefore absorb less light than those at the periphery. Effectively, when distributed homogeneously within the tissue, fewer red blood cells would produce the same absorption as the actual number of red blood cells distributed in discrete vessels. The correction factor can be described as

$$c(\lambda) = \frac{1-\exp(-2R\mu_a^{Blood}(\lambda))}{2R\mu_a^{Blood}(\lambda)} \qquad (Eq.4),$$

where R denotes the average vessel radius expressed in cm. The absorption coefficient related to blood is given by

$$\mu_a^{Blood}(\lambda) = \alpha_{BL}\mu_a^{HbO_2}(\lambda) + (1-\alpha_{BL})\mu_a^{Hb}(\lambda)\left[cm^{-1}\right] \qquad (Eq.5),$$

where $\mu_a^{HbO_2}(\lambda)$ and $\mu_a^{Hb}(\lambda)$ represent the basic extinction coefficient spectra of oxygenated hemoglobin $HbO_2$ and deoxygenated hemoglobin $Hb$, respectively. The oxygenated hemoglobin fraction in the total amount of hemoglobin is noted $\alpha_{EL} = [HbO_2]/([HbO_2] + [Hb])$ and is commonly known as the blood oxygen saturation. The absorption due to the presence of water and lipid in the measured tissue is defined as

$$\mu_a^{WL}(\lambda) = \alpha_{WL}\mu_a^{Lipid}(\lambda) + (1-\alpha_{WL})\mu_a^{H_2O}(\lambda)\left[cm^{-1}\right] \qquad (Eq.6)$$

In this case the concentration of lipid related to the total concentration of lipid and water together can be written as $\alpha_{WF} = [Lipid]/([Lipid] + [H_2O])$, where $[Lipid]$ and $[H_2O]$, correspond to the concentration of lipid (density of 0.86g/ml) and water, respectively.

This way of relating the water and lipid parameters in the expression of the absorption coefficient defined in Eq.6, rather than estimating separately the water and lipid volume fraction corresponds to a minimization of the covariance of the basic functions for fitting resulting in a more stable fit cf. the reference [2]. For further explanation and validation of this theorem reference is made to the reference Nachabe et al. JBO, 2010 [4], which is hereby included by reference in entirety.

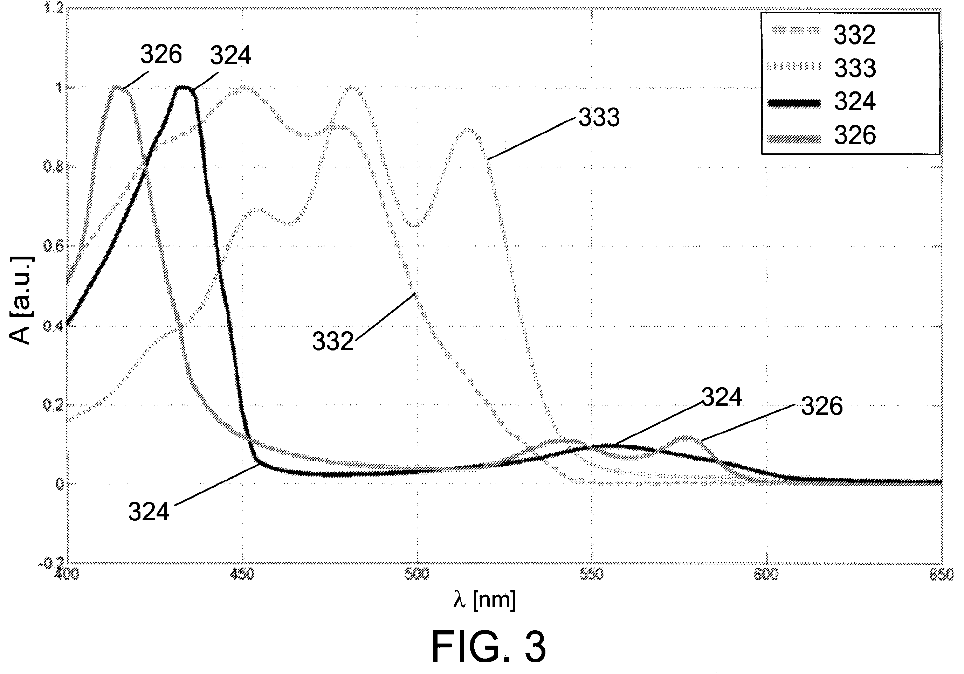

FIG 3 shows normalized absorption coefficients of beta-carotene 332, lycopene 333, deoxygenated haemoglobin (Hb) 324 and oxygenated haemoglobin (HbO2) 326 between 400 and 650 nm. The shown spectrum of lycopene has been measured by the present inventors. The graph has on its first, horizontal axis, the wavelength ($\square$, lambda) given in nanometer (nm), and on its second, vertical axis, the normalized absorption (A) given in arbitrary units (a.u.).

In FIG 3 we show the absorption curves of beta-Carotene as well as lycopene that we have measured. When the absorption by lycopene and beta-carotene is included into the model we should replace Eq. 3 by

$$\mu_a^{Tissue}(\lambda) = C(\lambda)\upsilon_{Blood}\,\mu_a^{Blood}(\lambda) + \upsilon_{WL}\,\mu_a^{WL}(\lambda) + \upsilon_{Lyco}\mu_a^{Lyco}(\lambda) + \upsilon_{BC}\mu_a^{BC}(\lambda)\,[cm^{-1}] \qquad (Eq.7),$$

where $v_{Lyco}$ and $v_{EC}$ are the volume fractions, while $\mu_a^{Lyco}$ and $\mu_a^{BC}$ represent the absorption coefficients of lycopene and β-carotene, respectively.

FIG 4 shows a DRS measurement of normal prostatic tissue and fitting with standard chromophores (range: 400-1600 nm), i.e., a typical DRS spectrum from 400 to 1600 nm of a associated tissue, being a normal prostatic tissue sample (shown as the light grey curve 340 composed of individual measured points), the corresponding fit curve with the "default" chromophores (blood, water, fat and scattering) added into the model (shown as the dark curve 342 which is continuous). The dotted black line 338 indicates the residual, which is the difference between the blue and red line.

When investigating the residual, a large deviation between the measurement and the fit curves is observed in the region 400-600 nm and at the peaks near 960 and 1200 nm. This deviation generally indicates that a chromophore is missing in the fitting model, which can induce under- and overestimation of other parameters.

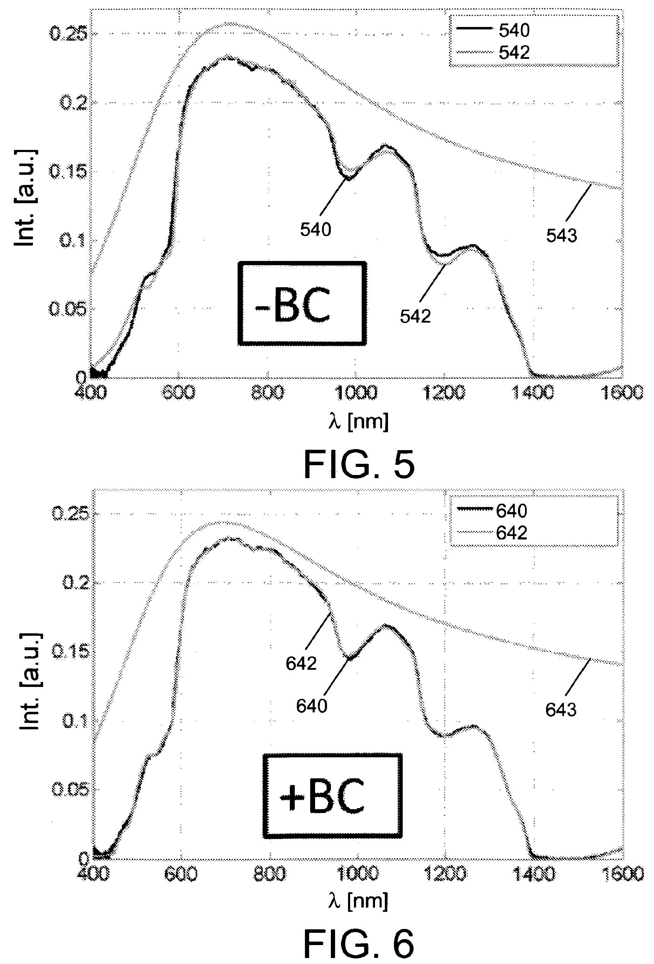

FIG 5 shows the result of fitting with only default chromophores (i.e., without beta-carotene, "-BC"). In the figure, the measured data are shown as the dark curve 540, while the fit is shown as the light grey curve 542. The upper curve 543 shows the calculated contribution to the spectrum of scattering only, i.e. the way the spectrum would be in absence of absorption hence scattering only.

FIG 6 shows the result of fitting wherein the fit includes beta-carotene ("+BC") which yields much better fit results, as can be observed by the fact that the dark curve 640 which is representative of the measured data throughout the depicted range of 400-1600 nm is very close to the light grey curve 642 which is the fit. The upper curve 643 shows the calculated contribution to the spectrum of scattering only i.e. the way the spectrum would be in absence of absorption hence scattering only.

I.e., FIGS 5-6 show that when adding beta-Carotene to the fit model a significant improvement is observed.

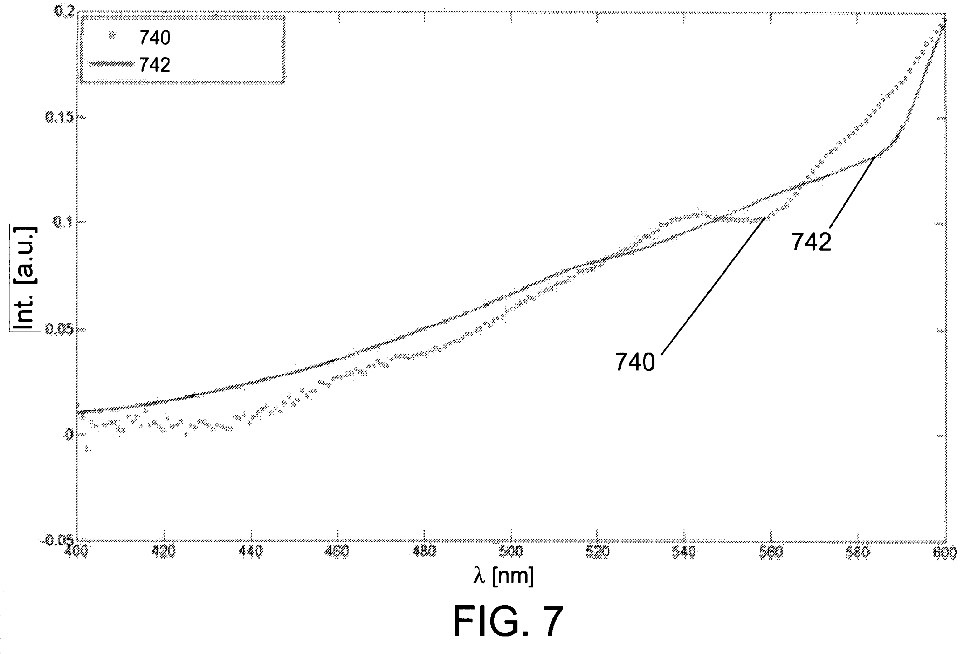

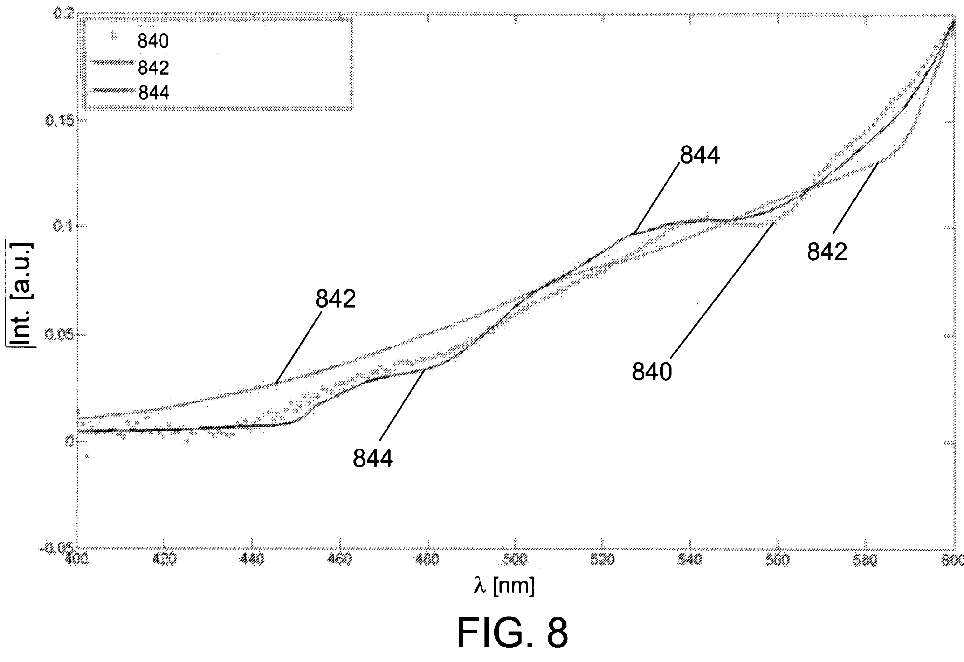

When zooming in on the region 400-600 nm, it is observed indeed that the fit significantly improves (see FIG 7 and FIG 8).

FIG 7 shows a standard fit (i.e., with only the default chromophores not including neither carotenoids) for a wavelength range of 400-600 nm, where the measured data are shown as the individual dots forming curve 740, while the fit is shown as the continuous curve 742. FIG 7 corresponds to a zoom of FIG 5.

FIG 8 shows a fit with β-carotene (beta-carotene) included into the model for a wavelength range of 400-600 nm, where the measured data are shown as the individual dots forming curve 840, the "default fit" with only the default chromophores is shown as the light grey continuous curve 842 and the dark continuous curve 844 shows the result of fitting wherein the fit includes beta-carotene which yields much better fit results, as can be observed by the fact that almost throughout the depicted range, the measured data points 840 are closer to the dark grey curve 844 which is the fit including beta-carotenoid than the default fit 842.

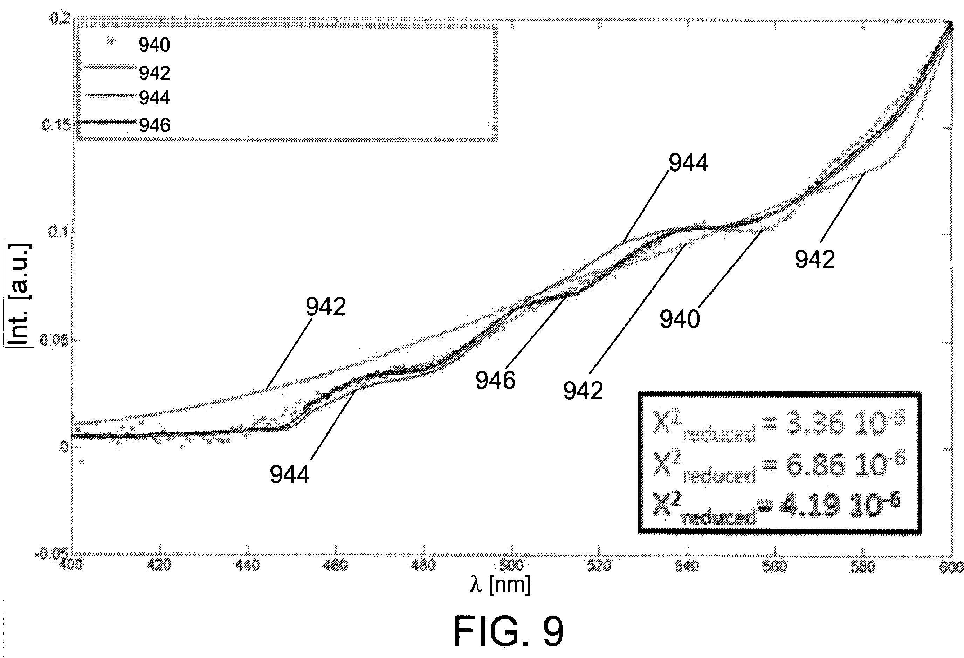

FIG 9 shows a fit with β-carotene (beta-carotene) and lycopene included into the model for a wavelength range of 400-600 nm, where the measured data are again shown as the individual dots forming curve 940, the "default fit" with only the default chromophores is shown as the light grey continuous curve 942 and the darker continuous curve 944 shows the result of fitting wherein the fit includes beta-carotene, and the darkest continuous curve 946 shows the result of fitting wherein the fit includes both beta-carotene and lycopene in the fitting model. The latter (i.e., fitting with both beta-carotene and lycopene) yields better the best fit results, as can be observed by the fact that the measured data points 946 are closest to the darkest grey curve 946 which is the fit including beta-carotenoid and lycopene.

Furthermore, the $\chi^2$ (chi-squared) values, which may be taken as a measure of how well the fit approaches the measured data, for the three fits are, respectively, given by:

$\chi^2 = 3.36 * 10^{-5}$ (for the default fit corresponding to curve 942),
$\chi^2 = 6.86 * 10^{-6}$ (for the fit with beta-carotene corresponding to curve 944),

$\chi^2 = 4.19 * 10^{-6}$ (for the fit with beta-carotene and lycopene corresponding to curve 946).

From the chi-squared values, it can thus be seen that adding beta-carotene improves the fit, and furthermore that addition of both beta-carotene and lycopene improves the fit further.

To sum up on the information which can be derived from FIGS 4-9: When adding beta-carotene to the model it is indeed observed that the fit significantly improves (see FIG 7 and FIG 8). Furthermore, by adding lycopene as well to the model the fit further significantly improves (see FIG 9).

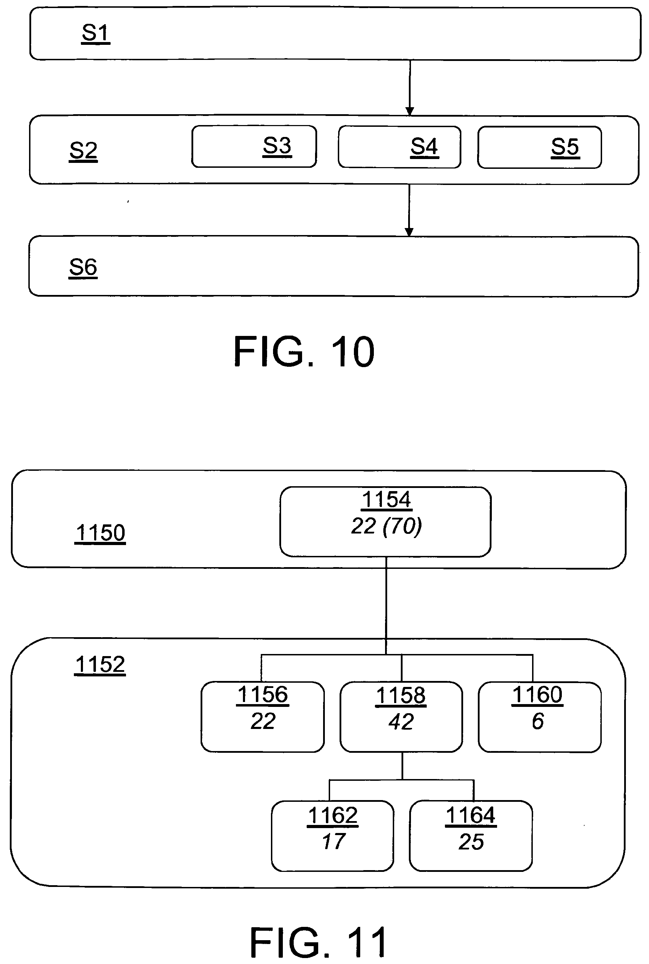

FIG 10 is a flowchart of a method according to the invention comprising the steps of measuring S1 data representative of an optical spectrum of the associated tissue by emitting and receiving photons via an interventional device, determination S2 of a first parameter, the first parameter being indicative of a concentration of lycopene, based on the optical spectrum, wherein the determination of the first parameter includes any one of: fitting S3 the measured data to a mathematical model, accessing S4 a look-up-table comprising one or more predetermined optical spectra, and performing S5 multivariate analysis. The method further comprising determination S6 of a second parameter based on the first parameter, the second parameter being indicative of a tissue type.

In a particular embodiment, diffuse reflectance spectroscopy is used for obtaining measured data representative of an optical spectrum. Although diffuse reflectance spectroscopy (DRS) is described to extract tissue properties also other optical methods can be envisioned, such as fluorescence spectroscopy measurements, diffuse optical tomography by employing a plurality of optical fibers, or differential path length spectroscopy (DPS). It is noted that in a particular embodiment is preferred not to use an apparatus which is arranged for obtaining measured data representative of the optical spectrum, wherein the optical spectrum is a Raman Spectrum.

The measurement of the optical spectrum can be carried out in various ways, such as by means of various filter systems in different positions of the optical path, one or more light sources emitting in different wavelength bands, or detectors for different wavelength bands. This is understood to be commonly known by the skilled person. It is also possible to modulate the various wavelength bands with different modulation frequencies at the source and demodulate these at the detector, (this technique is described the published patent application WO2009/153719 which is hereby incorporated by reference in its entirety).

Various other modifications can be envisioned without departing from the scope of the invention for instance using more than one detector or using more than one light source with different wavelength band, such as Light Emitting Diodes (LEDs) or laser sources.

Determining the second parameter indicative of tissue type

A total of 27 prostates were measured in connection with the studies for the present application.

TABLE I (in ANNEX I appended at the end of the present description) provides an overview of measured prostates with corresponding patient year of birth, main Gleason score and number of biopsies used for analysis. In total 5 prostate samples were excluded from the analysis because of missing useful biopsies samples that were taken at the location where the optical measurement was performed. Hence no pathology information was available for these 5 prostates at the optical measurement locations.

FIG 11 shows an overview of classification of associated tissue samples.

Block 1150 represents "prostates", and in block 1154 it is noted that 22 prostates were measured (and in parenthesis it is indicated that 70 biopsies were taken).

The measurements were classified as "tumor" (>25% tumor), "glandular" (>25% glandular, 0% tumor) or "fibromuscular" (<25% glandular, 0% tumor).

Block 1152 represents "biopsies", and in block 1156 it is indicated that 22 biopsies are classified as "tumor", in block 1158 it is indicated that 42 biopsies are classified as "non-tumor", in block 1156 it is indicated that 6 biopsies are classified as "mixed", in block 1162 it is indicated that (of the 42 "non-tumor" biopsies) 17 biopsies are classified as "glandular", in block 1164 it is indicated that (of the 42 "non-tumor" biopsies) 25 biopsies are classified as "fibromuscular".

Lycopene is particularly interesting, since it can be used to discriminate normal prostate tissue from tumor prostate tissue, hence can be used to determine tissue types, i.e., by determining a concentration of lycopene, a second parameter indicative of tissue type may be determined.

In an article of Clinton *et al*. [5], which is hereby incorporated by reference in entirety, the presence of various carotenoids in the prostate have been investigated in 25 men, aged 53 to 74. A high performance liquid chromatography system was used to determine and quantify the concentrations of lycopene, β-carotene (beta-carotene), and other carotenoids in both normal and tumor tissue. Clinton *et al*. [5] concluded that a diverse array of carotenoids is present in the human prostate. Lycopene and beta-carotene appeared to be the predominant carotenoids observed, with means ± SD of 0.80 ± 0.08 nmol/g and 0.54 ± 0.09 nmol/g, respectively. Together, this is more than half of the total concentration of carotenoids present in the prostate.

TABLE II (in ANNEX I appended at the end of the present description) shows results from ref. [5], i.e., Clinton et al., Cancer Epidemiology Biomarkers Prevention, vol. 5, 1996; Comparison of carotenoid concentrations in paired "normal" and "cancer" tissue from prostatectomy specimens from 25 men treated for localized prostate cancer (The asterisk (*) indicates that the difference between "normal" and "cancer" was significant for the isomer all-trans beta-carotene, but not for the isomer 9-cis beta-carotene).

Results averaged over all patients

TABLE III (in ANNEX I appended at the end of the present description) shows a comparison of averaged fit results (i.e., mean and standard deviation) of normal tissue and tumor (the asterisk (*) indicates significant differences with $p < 0.01$ for the Kruskal-Wallis test). The blood oxygenation is not shown, due to the fact that the study was conducted on ex vivo samples, i.e. ex vivo associated tissue samples. Therefore this parameter does not reflect the actual oxygenation level as it would be in vivo. Since the data did not follow a Gaussian distribution, not a T-test, but the non-parametric statistical Kruskal-Wallis test was used to find which parameters were significantly different in tumor lesions compared to either glandular, stroma or normal tissue. According to the Kruskal-Wallis test ($p < 0.01$), show that both beta-carotene and lycopene show significant differences with $p < 0.01$. When we compare the concentrations found for beta-carotene and lycopene and compare them to Clinton et al. [5] we find that they compare well. Furthermore, these results show that both beta-carotene and lycopene can be used as a discriminating factor for distinguishing between various tissue types, and in particular for distinguishing between normal and tumor tissue in prostate.

Applications of the invention

Particular embodiments of the invention may be used in the field surgery or minimally invasive interventional diagnosis and/or treatment within organs, such as within the prostate organ or related structures.

Particular embodiments of the invention may be used in the field of oncology, or other healthcare applications where the determination of tissue type is relevant.

The apparatus may be applicable for real-time intra-operative needle localization and ablation monitoring to improve ablation efficacy and disease free survival.

To sum up, the present invention relates to an apparatus 100 and, a method and a computer program for determining a first parameter indicative of a concentration of lycopene. In particular, the invention relates to an apparatus 100 comprising a spectrometer 102, which spectrometer comprises a light source 104 and a detector 106, 108 arranged to measure an optical spectrum via an interventional device 112. This enables determination of a first parameter being indicative of a lycopene concentration. Lycopene concentration may serve as a discriminative feature for different tissue types, such as the prostate organ and associated structures. The apparatus may in a specific embodiment be arranged to determine a second parameter indicative of a tissue type based on a concentration of lycopene. According to a specific embodiment, the apparatus relies on Diffuse Reflectance Spectroscopy (DRS).

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

References

[1] T.J. Farrel, M.S. Patterson and B.C. Wilson, "A diffusion theory model of spatially resolved, steady-state diffuse reflectance for the non-invasive determination of tissue optical properties," Med. Phys. 19 (1992) p. 879-888.

[2] R. Nachabé, B.H.W. Hendriks, M.V.D. Voort, A. E, and H.J.C.M. Sterenborg, "Estimation of biological chromophores using diffuse optical spectroscopy : benefit of extending the UV-VIS wavelength range to include 1000 to 1600 nm," Optics Express, vol. 18, 2010, pp. 879-888.

[3] R. Nachabé, D.J. Evers, B.H.W. Hendriks, G.W. Lucassen, M.V.D. Voort, J. Wesseling, and T.J.M. Ruers, "Effect of bile absorption coefficients on the estimation of liver tissue optical properties and related implications in discriminating healthy and tumorous samples," Biomedical optics express, vol. 2, 2011, pp. 600-614.

[4] R. Nachabe, B.H.W. Hendriks, A.E. Desjardins, M. van der Voort, M.B. van der Mark, and H.J.C.M. Sterenborg, "Estimation of lipid and water concentrations in scattering media with diffuse optical spectroscopy from 900 to 1600 nm," Journal of biomedical Optics, vol. 15, May. 2010, pp. 037015-10.

[5] S.K. Clinton, C. Emenhiser, S.J. Schwartz, D.G. Bostwick, A.W. Williams, B.J. Moore, and J. Erdman, "cis-trans lycopene isomers, carotenoids, and retinol in the human prostate.," Cancer Epidemiology Biomarkers & Prevention, vol. 5, 1996, p. 823.

ANNEX I

TABLE I

| Prostate # | Patient year born | Main Gleason score | Number of useful biopsies |
|---|---|---|---|
| PR1 | 1940 | 3+3=6 | 0 |
| PR2 | 1968 | 3+3=6 | 0 |
| PR3 | 1947 | 3+3=6 | 4 |
| PR4 | 1945 | 3+3=6 | 4 |
| PR5 | 1951 | 3+3=6 | 0 |
| PR6 | 1944 | 3+3=6 | 0 |
| PR7 | 1954 | 3+4=7 | 0 |
| PR8 | 1949 | 3+3=6 | 3 |
| PR9 | 1950 | 3+3=6 | 3 |
| PR10 | 1955 | 3+3=6 | 3 |
| PR11 | 1947 | 3+3=6 | 2 |
| PR12 | 1945 | 3+3=6 | 4 |
| PR13 | 1943 | 4+3=7 | 3 |
| PR14 | 1956 | 3+4=7 | 3 |
| PR15 | 1947 | 3+3=6 | 3 |
| PR16 | 1954 | 3+3=6 | 3 |
| PR17 | 1945 | 3+3=6 | 3 |
| PR18 | 1944 | 4+4=8 | 4 |
| PR19 | 1952 | 3+3=6 | 3 |
| PR20 | 1944 | 3+4=7 | 2 |
| PR21 | 1955 | 3+3=6 | 4 |
| PR22 | 1947 | 3+4=7 | 3 |
| PR23 | 1949 | 3+3=6 | 3 |
| PR24 | 1943 | 3+4=7 | 4 |
| PR25 | 1950 | 3+4=7 | 3 |
| PR26 | 1945 | 3+4=7 | 3 |
| PR27 | 1938 | 4+3=7 | 3 |
| | Average: 1948 | Most frequent: 3+3=6 | Total: 70 |

TABLE II

| | Normal(nmol/g) mean $\pm$ SE | Cancer (nmol/g) mean $\pm$ SE | Pairwise comparison |
|---|---|---|---|
| $\beta$-Carotene | $0.86 \pm 0.06$ | $1.00 \pm 0.08$ | P<0.02 / NS-* |
| Lycopene | $0.63 \pm 0.09$ | $0.91 \pm 0.13$ | P <0.03 |
| $\beta$-Carotene | $0.35 \pm 0.06$ | $0.35 \pm 0.05$ | NS |
| Lutein | $0.26 \pm 0.05$ | $0.33 \pm 0.05$ | NS |
| $\alpha$-Cryptoxanthin | $0.22 \pm 0.03$ | $0.29 \pm 0.03$ | NS |
| Zeaxanthin | $0.19 \pm 0.04$ | $0.29 \pm 0.06$ | NS |
| $\beta$-Cryptoxanthin | $0.14 \pm 0.02$ | $0.18 \pm 0.03$ | NS |
| Total Carotenoids | $2.65 \pm 0.25$ | $3.35 \pm 0.32$ | P <0.02 |

**TABLE III**

| DRS parameters | NORMAL (N) | TUMOR (T) | P-VALUE<br>N vs T |
|---|---|---|---|
| Blood volume fraction(%) | 0.64 ± 0.78 | 0.51 ± 0.55 | 0.483 |
| Average vessel radius (microns) | 38 ± 110 | 64 ± 165 | 0.997 |
| Water volume fraction (%) | 100 ± 18 | 98 ± 16 | 0.000* |
| Lipid volume fraction (%) | 4.0 ± 4.8 | 3.1 ± 3.1 | 0.011 |
| Lycopene (μM) | 1.2 ± 2.4 | 2.2 ± 3.8 | 0.000* |
| β-carotene (μM) | 1.6 ± 1.1 | 2.2 ± 1.4 | 0.000* |
| Reduced scattering at 800 nm (cm-1) | 11.0 ± 2.6 | 11.7 ± 4.3 | 0.000* |
| Mie slope | 0.42 ± 0.21 | 0.45 ± 0.50 | 0.004* |
| Mie-to-Rayleigh scattering fraction (%) | 0.56 ± 0.12 | 0.56 ± 0.13 | 0.336 |
| *FS parameters* | | | |
| Collagen+Elastin relative fluorescent intensity (%) | 66 ± 23 | 73 ± 14 | 0.000* |
| NADH relative fluorescent intensity (%) | 30 ± 23 | 21 ± 12 | 0.000* |
| FAD relative fluorescent intensity (%) | 4.9 ± 2.6 | 5.4 ± 2.8 | 0.003* |
| Total fluorescent intensity * 100 000 (a.u.) | 2.3 ± 1.2 | 2.7 ± 1.3 | 0.000* |

**Claims**

1. An apparatus (100) for optical analysis of an associated tissue (116), the apparatus comprising:

- a spectrometer (102) for obtaining measured data representative of an optical spectrum of the associated tissue (116), wherein the optical spectrum is a Diffuse Reflectance Spectrum, the spectrometer comprising

- a light source (104),
- an optical detector (106),

an interventional device (112) suitable for insertion into the associated tissue (116), the interventional device comprising

- one or more guides (217, 219) for

- guiding photons from the light source (104) to an exit position (218) on a distal end of the interventional device, the photons being emittable from the exit position, and for
- guiding photons from an entry position (220) on the distal end of the interventional device and to the optical detector (106),

so as to enable obtaining the measured data representative of the optical spectrum of the associated tissue (116) at a position of the distal end of the interventional device, and

- a processor (110) arranged for

- receiving the measured data,
- based on a lycopene specific absorption profile, determining from the measured data a first parameter being indicative of a concentration of lycopene in said associated tissue (116),

wherein the processor (110) is further arranged for determining from the first parameter a second parameter being indicative of a tissue type.

2. An apparatus (100) according to claim 1, wherein the exit position (218) and the entry position (220) being positioned with sufficient relative distance for Diffusive Reflectance Spectrometry to be performed.

**3.** An apparatus according to claim 1, wherein the processor (110) is further arranged for determining one or more third parameters, wherein each of the one or more third parameters is indicative of a concentration of a chromophore other than lycopene in said associated tissue (116).

**4.** An apparatus according to claim 1, wherein the processor (110) is further arranged for determining a scattering parameter based on the measured data.

**5.** An apparatus according to claim 1, wherein the one or more guides comprising

- a first guide (217) for guiding photons from the light source (104) to an exit position (218) on a distal end of the interventional device, the photons being emittable from the exit position, and
- a second guide (219) for guiding photons from an entry position (220) on the distal end of the interventional device and to the optical detector (106).

**6.** An apparatus (100) according to claim 1, wherein the exit position (218) and the entry position (220) are spatially separated and spatially oriented so that, upon positioning the distal end of the interventional device (112) adjacent to the associated tissue (116) the photons emittable at the exit position (218) and subsequently collectable at the entry position (220) are diffusive photons which experience multiple scattering events.

**7.** An apparatus (100) according to claim 1, wherein the exit position (218) and the entry position (220) are spatially separated and spatially oriented so that, upon positioning the distal end of the interventional device (112) adjacent to the associated tissue (116), the entry position is not intersected by ballistic photons emitted from the exit position, when the distal end of the interventional device is placed adjacent the associated sample.

**8.** An apparatus according to claim 1, wherein the apparatus further comprises a database (114), which database is operably connected to the processor (110).

**9.** An apparatus according to claim 8, wherein the database (114) comprises predetermined data representative of one or more optical spectra, wherein one predetermined data is representative of an optical spectrum of lycopene.

**10.** A method for optical analysis of an associated tissue, the method comprising the steps of:

- measuring (S1) data representative of an optical spectrum of the associated tissue by emitting and receiving photons via an interventional device, wherein the optical spectrum is a Diffuse Reflectance Spectrum,
- based on a lycopene specific absorption profile, determining (S2) a first parameter, the first parameter being indicative of a concentration of lycopene, based on the optical spectrum,
- determining (S6) a second parameter based on the first parameter, the second parameter being indicative of a tissue type.

**11.** A method according to claim 10 for optical analysis of an associated tissue, wherein the determination of the first parameter includes any one of:

- fitting (S3) the measured data to a mathematical model,
- accessing (S4) a look-up-table comprising one or more predetermined optical spectra, and
- performing (S5) multivariate analysis.

**12.** A computer program product being adapted to enable a computer system comprising at least one computer having data storage means associated therewith to operate a processor arranged for carrying out the method according to claim 10.

**Patentansprüche**

**1.** Vorrichtung (100) zur optischen Analyse eines zugehörigen Gewebes (116), wobei die Vorrichtung Folgendes umfasst:

- ein Spektrometer (102) zum Erlangen von Messdaten, die für ein optisches Spektrum des zugehörigen Gewebes (116) repräsentativ sind, wobei das optische Spektrum ein diffuses Reflexionsspektrum ist, wobei das Spektrometer Folgendes umfasst:

    - eine Lichtquelle (104),
    - einen optischen Detektor (106),
    - ein interventionelles Instrument (112), das zur Einführung in das zugehörige Gewebe (116) geeignet ist, wobei das interventionelle Instrument Folgendes umfasst:
    - eine oder mehrere Führungen (217, 219) zum

        - Führen von Photonen von der Lichtquelle (104) zu einer Austrittsposition (218) an einem distalen Ende des interventionellen Instruments, wobei die Photonen von der Austrittsposition emittierbar sind, und zum
        - Führen von Photonen von einer Eintrittsposition (220) am distalen Ende des interventionellen Instruments und zu dem optischen Detektor (106),

    um so das Erlangen der Messdaten zu ermöglichen, die für das optische Spektrum des zugehörigen Gewebes (116) an der Position des distalen Endes des interventionellen Instruments repräsentativ sind, und

    - einen Prozessor (110), der ausgelegt ist zum

        - Empfangen der Messdaten,
        - basierend auf einem Lycopin-spezifischen Absorptionsprofil Ermitteln eines ersten Parameters anhand der Messdaten, der eine Konzentration von Lycopin in dem genannten zugehörigen Gewebe (116) angibt,

    wobei der Prozessor (110) weiterhin dafür ausgelegt ist, anhand des ersten Parameters einen zweiten Parameter zu ermitteln, der einen Gewebetyp angibt.

2.  Vorrichtung (100) nach Anspruch 1, wobei die Austrittsposition (218) und die Eintrittsposition (220) mit ausreichendem relativen Abstand für die Durchführung der diffusen Reflexionsspektrometrie positioniert sind.

3.  Vorrichtung nach Anspruch 1, wobei der Prozessor (110) weiterhin dafür ausgelegt ist, einen oder mehrere dritte Parameter zu ermitteln, wobei jeder der einen oder mehreren dritten Parameter eine Konzentration eines anderen Chromophoren als Lycopin in dem genannten zugehörigen Gewebe (116) angibt.

4.  Vorrichtung nach Anspruch 1, wobei der Prozessor (110) weiterhin dafür ausgelegt ist, basierend auf den Messdaten einen Streuparameter zu ermitteln.

5.  Vorrichtung nach Anspruch 1, wobei die eine oder mehrere Führungen Folgendes umfassen:

    - eine erste Führung (217) zum Führen der Photonen von der Lichtquelle (104) zu einer Austrittsposition (218) an einem distalen Ende des interventionellen Instruments, wobei die Photonen von der Austrittsposition aus emittierbar sind, und
    - eine zweite Führung (219) zum Führen der Photonen von einer Eintrittsposition (220) an einem distalen Ende des interventionellen Instruments und zu dem optischen Detektor (106).

6.  Vorrichtung (100) nach Anspruch 1, wobei die Austrittsposition (218) und die Eintrittsposition (220) räumlich getrennt und räumlich so ausgerichtet sind, dass, nach dem Positionieren des distalen Endes des interventionellen Instruments (112) angrenzend an das zugehörige Gewebe (116), die an der Austrittsposition (218) emittierbaren und anschließend an der Eintrittsposition (220) sammelbaren Photonen diffuse Photonen sind, welche mehrere Streuereignisse erfahren.

7.  Vorrichtung (100) nach Anspruch 1, wobei die Austrittsposition (218) und die Eintrittsposition (220) räumlich getrennt und räumlich so ausgerichtet sind, dass, nach dem Positionieren des distalen Endes des interventionellen Instruments (112) angrenzend an das zugehörige Gewebe (116), die Eintrittsposition nicht durch von der Austrittsposition emittierte ballistische Photonen geschnitten wird, wenn das distale Ende des interventionellen Instruments angrenzend an die zugehörige Probe platziert ist.

8.  Vorrichtung nach Anspruch 1, wobei die Vorrichtung weiterhin eine Datenbank (114) umfasst, die betriebsfähig mit dem Prozessor (110) verbunden ist.

9.  Vorrichtung nach Anspruch 8, wobei die Datenbank (114) vorgegebene Daten umfasst, die für ein oder mehrere optische Spektren repräsentativ sind, wobei ein vorgegebenes Datenelement für ein optisches Spektrum von Lycopin repräsentativ ist.

10. Verfahren zur optischen Analyse eines zugehörigen Gewebes, wobei das Verfahren die folgenden Schritte umfasst:

    - Messen (S1) von Daten, die für ein optisches Spektrum des zugehörigen Gewebes repräsentativ sind, durch Emittieren und Empfangen von Photonen über ein interventionelles Instrument, wobei das optische Spektrum ein diffuses Reflexionsspektrum ist,
    - basierend auf einem Lycopin-spezifischen Absorptionsprofil Ermitteln (S2) eines ersten Parameters, wobei der erste Parameter eine Konzentration von Lycopin angibt, basierend auf dem optischen Spektrum,
    - Ermitteln (S6) eines zweiten Parameters basierend auf dem ersten Parameter, wobei der zweite Parameter einen Gewebetyp angibt.

11. Verfahren nach Anspruch 10 zur optischen Analyse eines zugehörigen Gewebes, wobei das Ermitteln des ersten Parameters eines von Folgendem umfasst:

    - Anpassen (S3) der Messdaten an ein mathematisches Modell,
    - Zugreifen (S4) auf eine Nachschlagetabelle umfassend ein oder mehrere vorgegebene optische Spektren, und
    - Durchführen (S5) einer multivariaten Analyse.

12. Computerprogrammprodukt, das dafür ausgelegt ist, einem Computersystem umfassend mindestens einen Computer mit zugehörigen Datenspeichermitteln zu ermöglichen, einen Prozessor zu betreiben, der dafür ausgelegt ist, das Verfahren nach Anspruch 10 durchzuführen.

**Revendications**

1.  Appareil (100) pour analyse optique d'un tissu associé (116), l'appareil comprenant :

    - un spectromètre (102) pour obtenir des données mesurées représentatives d'un spectre optique du tissu associé (116), dans lequel le spectre optique est un spectre à facteur de réflexion diffuse, le spectromètre comprenant

        - une source de lumière (104),
        - un détecteur optique (106),
        - un dispositif d'intervention (112) approprié pour insertion dans le tissu associé (116), le dispositif d'intervention comprenant

            - un ou plusieurs guides (217, 219) pour
            - guider les photons depuis la source de lumière (104) jusqu'à une position de sortie (218) sur une extrémité distale du dispositif d'intervention, les photons pouvant être émis depuis la position de sortie, et pour
            - guider les photons depuis une position d'entrée (220) sur l'extrémité distale du dispositif d'intervention et jusqu'au détecteur optique (106),

        de manière à permettre l'obtention des données mesurées représentatives du spectre optique du tissu associé (116) dans une position de l'extrémité distale du dispositif d'intervention, et

    - un processeur (110) agencé pour

        - recevoir les données mesurées,
        - sur la base d'un profil d'absorption spécifique au lycopène, déterminer à partir des données mesurées un premier paramètre indicatif d'une concentration de lycopène dans ledit tissu associé (116),

dans lequel le processeur (110) est en outre agencé pour déterminer à partir du premier paramètre un deuxième paramètre indicatif d'un type de tissu.

2. Appareil (100) selon la revendication 1, dans lequel la position de sortie (218) et la position d'entrée (220) étant positionnées avec une distance relative suffisante pour la spectrométrie à facteur de réflexion diffuse devant être réalisée.

3. Appareil selon la revendication 1, dans lequel le processeur (110) est en outre agencé pour déterminer un ou plusieurs troisièmes paramètres, dans lequel chacun du ou des troisièmes paramètres est indicatif d'une concentration d'un chromophore autre que le lycopène dans ledit tissu associé (116).

4. Appareil selon la revendication 1, dans lequel le processeur (110) est en outre agencé pour déterminer un paramètre de dispersion sur la base des données mesurées.

5. Appareil selon la revendication 1, dans lequel le ou les guides comprennent

  - un premier guide (217) pour guider les photons depuis la source de lumière (104) jusqu'à une position de sortie (218) sur une extrémité distale du dispositif d'intervention, les photons pouvant être émis depuis la position de sortie, et
  - un deuxième guide (219) pour guider les photons depuis une position d'entrée (220) sur l'extrémité distale du dispositif d'intervention et jusqu'au détecteur optique (106).

6. Appareil (100) selon la revendication 1, dans lequel la position de sortie (218) et la position d'entrée (220) sont spatialement séparées et spatialement orientées de telle sorte que, lors du positionnement de l'extrémité distale du dispositif d'intervention (112) de manière adjacente au tissu associé (116), les photons pouvant être émis dans la position de sortie (218) et ensuite être collectés dans la position d'entrée (220) sont des photons diffus qui subissent de multiples événements de dispersion.

7. Appareil (100) selon la revendication 1, dans lequel la position de sortie (218) et la position d'entrée (220) sont spatialement séparées et spatialement orientées de telle sorte que, lors du positionnement de l'extrémité distale du dispositif d'intervention (112) de manière adjacente au tissu associé (116), la position d'entrée n'est pas coupée par les photons balistiques émis depuis la position de sortie, quand l'extrémité distale du dispositif d'intervention est placée de manière adjacente à l'échantillon associé.

8. Appareil selon la revendication 1, dans lequel l'appareil comprend en outre une base de données (114), laquelle base de données est fonctionnellement connectée au processeur (110).

9. Appareil selon la revendication 8, dans lequel la base de données (114) comprend des données prédéterminées représentatives d'un ou de plusieurs spectres optiques, dans lequel une donnée prédéterminée est représentative d'un spectre optique de lycopène.

10. Procédé pour analyse optique d'un tissu associé, le procédé comprenant les étapes consistant à :

  - mesurer (S1) des données représentatives d'un spectre optique du tissu associé en émettant et recevant des photons par l'intermédiaire d'un dispositif d'intervention, dans lequel le spectre optique est un spectre à facteur de réflexion diffuse,
  - sur la base d'un profil d'absorption spécifique au lycopène, déterminer (S2) un premier paramètre, le premier paramètre étant indicatif d'une concentration de lycopène, sur la base du spectre optique,
  - déterminer (S6) un deuxième paramètre sur la base du premier paramètre, le deuxième paramètre étant indicatif d'un type de tissu.

11. Procédé selon la revendication 10 pour analyse optique d'un tissu associé, dans lequel la détermination du premier paramètre inclut l'une quelconque des actions suivantes :

  - l'adaptation (S3) des données mesurées à un modèle mathématique,
  - l'accès (S4) à un tableau de conversion comprenant un ou plusieurs spectres optiques prédéterminés, et
  - la réalisation (S5) d'une analyse multivariée.

**12.** Produit de programme informatique adapté pour permettre à un système informatique comprenant au moins un ordinateur ayant un moyen de stockage de données associé à celui-ci de faire fonctionner un processeur agencé pour réaliser le procédé selon la revendication 10.

FIG. 1

FIG. 2

FIG. 3

EP 2 806 785 B1

λ [nm]

FIG. 4

FIG. 5

FIG. 6

FIG. 7

EP 2 806 785 B1

FIG. 8

EP 2 806 785 B1

**FIG. 9**

EP 2 806 785 B1

S1

S2　　　　　S3　　　　　S4　　　　　S5

S6

# FIG. 10

<u>1150</u>

<u>1154</u>
*22 (70)*

<u>1152</u>

<u>1156</u>
*22*

<u>1158</u>
*42*

<u>1160</u>
*6*

<u>1162</u>
*17*

<u>1164</u>
*25*

# FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010144081 A1
- WO 2009153719 A

### Non-patent literature cited in the description

- **NACHABE et al.** *JBO,* 2010
- **CLINTON et al.** *Cancer Epidemiology Biomarkers Prevention,* 1996, vol. 5
- **T.J. FARREL ; M.S. PATTERSON ; B.C. WILSON.** A diffusion theory model of spatially resolved, steady-state diffuse reflectance for the non-invasive determination of tissue optical properties. *Med. Phys.,* 1992, vol. 19, 879-888
- **R. NACHABÉ ; B.H.W. HENDRIKS ; M.V.D. VOORT ; A. E, AND H.J.C.M. STERENBORG.** Estimation of biological chromophores using diffuse optical spectroscopy : benefit of extending the UV-VIS wavelength range to include 1000 to 1600 nm. *Optics Express,* 2010, vol. 18, 879-888
- **R. NACHABÉ ; D.J. EVERS ; B.H.W. HENDRIKS ; G.W. LUCASSEN ; M.V.D. VOORT ; J. WESSELING ; T.J.M. RUERS.** Effect of bile absorption coefficients on the estimation of liver tissue optical properties and related implications in discriminating healthy and tumorous samples. *Biomedical optics express,* 2011, vol. 2, 600-614
- **R. NACHABE ; B.H.W. HENDRIKS ; A.E. DESJARDINS ; M. VAN DER VOORT ; M.B. VAN DER MARK ; H.J.C.M. STERENBORG.** Estimation of lipid and water concentrations in scattering media with diffuse optical spectroscopy from 900 to 1600 nm. *Journal of biomedical Optics,* May 2010, vol. 15, 037015-10
- **S.K. CLINTON ; C. EMENHISER ; S.J. SCHWARTZ ; D.G. BOSTWICK ; A.W. WILLIAMS ; B.J. MOORE ; J. ERDMAN.** cis-trans lycopene isomers, carotenoids, and retinol in the human prostate. *Cancer Epidemiology Biomarkers & Prevention,* 1996, vol. 5, 823